(19) Europäisches Patentamt
European Patent Office
Office européen des brevets

(11) Veröffentlichungsnummer: **0 115 318**
**B1**

# (12) EUROPÄISCHE PATENTSCHRIFT

(45) Veröffentlichungstag der Patentschrift: **09.04.86**

(21) Anmeldenummer: **84100687.7**

(22) Anmeldetag: **24.01.84**

(51) Int. Cl.⁴: **C 07 F 9/09**, C 07 F 9/165, C 07 F 9/24, C 07 F 9/40, A 01 N 57/16, A 01 N 57/32, A 01 N 57/24

## (54) Oximinophosphorsäurederivate, Verfahren zu ihrer Herstellung und ihre Verwendung zur Bekämpfung von Schädlingen.

(30) Priorität: **29.01.83 DE 3302969**

(43) Veröffentlichungstag der Anmeldung: **08.08.84 Patentblatt 84/32**

(45) Bekanntmachung des Hinweises auf die Patenterteilung: **09.04.86 Patentblatt 86/15**

(84) Benannte Vertragsstaaten: **BE CH DE FR GB IT LI NL**

(56) Entgegenhaltungen: **EP - A - 0 031 574**
**DE - A - 2 262 189**

(73) Patentinhaber: **BASF Aktiengesellschaft, Carl-Bosch-Strasse 38, D-6700 Ludwigshafen (DE)**

(72) Erfinder: **Buerstinghaus, Rainer, Dr., Weinbergstrasse 85, D-6940 Weinheim (DE)**
Erfinder: **Himmele, Walter, Dr., Eichenweg 14, D-6909 Walldorf (DE)**
Erfinder: **Kiehs, Karl, Dr., Sudetenstrasse 22, D-6840 Lampertheim 1 (DE)**
Erfinder: **Adolphi, Heinrich, Dr., Kalmitweg 11, D-6703 Limburgerhof (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 115 318 B1

LIBER, STOCKHOLM 1986

## Beschreibung

Die vorliegende Erfindung betrifft Oximinophosphorsäurederivate, Verfahren zu ihrer Herstellung, Schädlingsbekämpfungsmittel, die diese Phosphorsäurederivate als Wirkstoffe enthalten, sowie ein Verfahren zur Bekämpfung von Schädlingen mit diesen Wirkstoffen.

Oximinophosphorsäurederivate sind aus der DE AS 10 52 981, der DE AS 12 38 902, der DE-OS 23 04 848 und der DE-OS 29 52 738 bekannt. Sie eignen sich zur Bekämpfung von Insekten und Spinnentieren. Ihre Wirkung ist jedoch speziell bei geringer Konzentration nicht immer ganz zufriedenstellend.

Es wurde gefunden daß Oximinophosporsäurederivate der Formel I

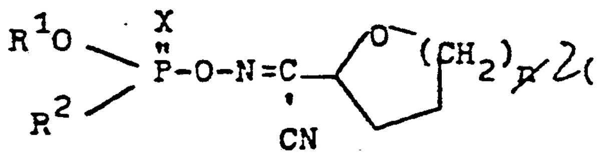
(I),

in der

$R^1$ eine unverzweigte oder verzweigte Alkylgruppe mit bis zu 4 Kohlenstoffatomen,

$R^2$ eine unverzweigte oder verzweigte Alkoxy- oder Alkylthiogruppe mit bis zu 4 Kohlenstoffatomen, eine unverzweigte oder verzweigte Alkylgruppe mit bis zu 3 Kohlenstoffatomen, Phenyl, die Aminogruppe oder einen unverzweigten oder verzweigten Alkylamino- oder Dialkylaminores mit jeweils bis zu 4 Kohlenstoffatomen in einer Alkylgruppe,

X Sauerstoff oder Schwefel bedeuten,

insektizid, akarizid und nematizid sehr gut wirksam und bekannten Wirkstoffen ähnlicher Struktur bzw. gleicher Wirkungsrichtung überlegen sind.

Die Oximinophosphorsäurederivate der Formel I können durch Umsetzung entsprechender α-Oximinonitrile mit entsprechenden (Thiono)(Thiol)phosphor(phosphon)säureester(amid)halogeniden erhalten werden:

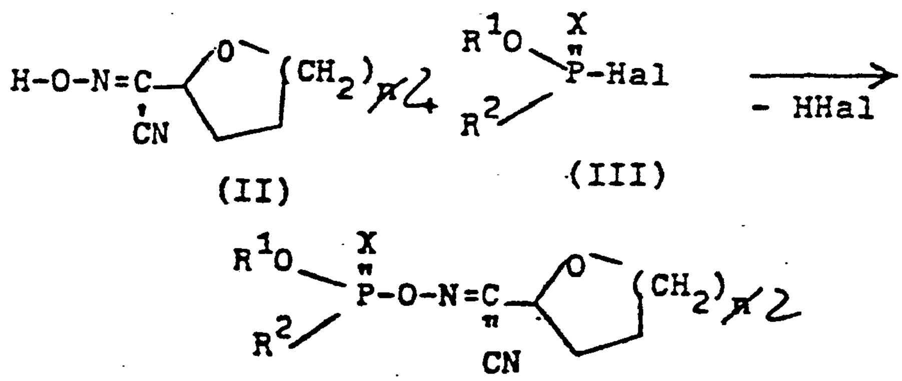

(II) (III)

(I)

Hal(ogen) bedeutet aus wirtschaftlichen Gründen vorzugsweise ein Chloratom.

Die Umsetzung wird zweckmäßigerweise in einem Lösungs- oder Verdünnungsmittel ausgeführt. Hierzu sind beispielsweise geeinet: aliphatische und aromatische, gegebenenfalls chlorierte Kohlenwasserstoffe, wie petrolether, Benzol, Toluol, Xylol, Benzin, Dichlormethan, Chloroform, Tetrachlormethan, 1,2-Dichlorethan, Chlorbenzol, Ether, wie Diethyl und Di-n-butylether, Methyl-tert.-butylether, Tetrahydrofuran, Dioxan; Ketone, beispielsweise Aceton, Methylethylketon, Methylisopropylketon; ferner Nitrile, wie Acetonitril und Propionitril. Auch Gemische dieser Stoffe können als Lösungs- oder Verdünnungsmittel verwendet werden.

Als Säurebindemittel (Säureacceptoren) eignen sich die bei der Phosphorylierung von Hydroxyverbindungen üblichen basischen Mittel. Besonders geeignet sind Alkalimetallcarbonate oder -alkoholate, wie Natrium- und Kaliumcarbonat, -methylat und -ethylat, ferner aliphatische, aromatische und heterocyclische Amine, z.B. Triethylamiu, Dimethylamin, Piperidin, Dimethylanilin, Dimethylbenzylamin und Pyridin. In einigen Fällen ist die Verwendung von Alkyllithiumverbindungen, z.B. n-Butyllithium oder Alkalimetallhydriden, z.B. Natriumhydrid vorteilhaft.

Anstelle des Zusatzes eines Säureacceptors kann man auch vor der Umsetzung die Salze der α-Oximinonitrile (II), etwa die Alkalimetall-, Erdalkalimetall- oder Ammoniumsalze herstellen und diese umsetzen.

Üblicherweise setzt man die Ausgangsstoffe in stöchiometrischem Verhältnis ein. Ein Überschuß des einen oder anderen kann in Einzelfällen aber durchaus vorteilhaft sein.

Die Umsetzung verläuft gewöhnlich oberhalb von Raumtemperatur mit ausreichender Geschwindigkeit. 120°C müssen i.a. nicht überschritten werden. Da sie in einigen Fällen unter Wärmeentwicklung verläuft, kann es von vorteil sein, eine Kühlmöglichkeit vorzusehen.

Das Reaktionsgemisch wird in üblicher Weise aufgearbeitet, z.B. durch Versetzen mit Wasser, Trennen der Phasen und Destillation und/oder Säulenchromatographie.

Die zur Herstellung der Verbindungen I als Ausgangsmaterialien verwendeten α-Oximinonitrile der Formel II sind neu. Sie lassen sich in an sich bekannter Weise (vgl. DE-AS 15 67 142) durch Chlorieren der zugehörigen 2-Formyltetrahydropyran-oxime der Formel IV und anschließende Umsetzung mit Natrium- oder Kaliumcyanid gemäß folgender Reaktionsgleichung herstellen:

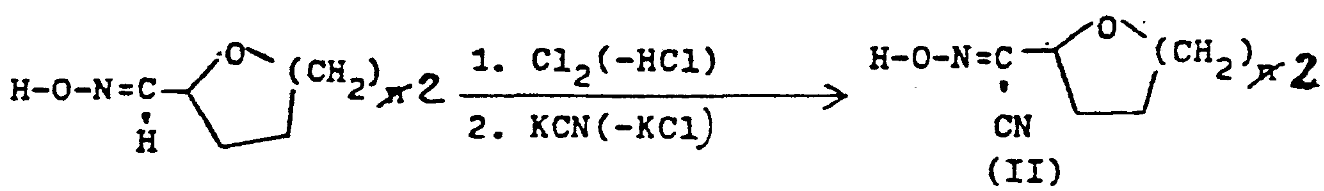


(IV) (II)

Oxime der Formel IV erhält man durch Umsetzen des betreffenden 2-Formyl-tetrahydropyrans der Formel V mit Hydroxylaminhydrochlorid nach folgender Reaktionsgleichung:

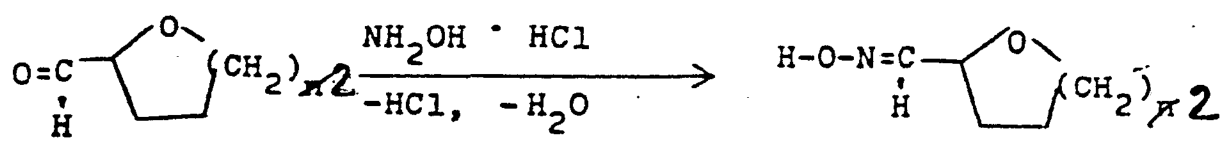


(V) (IV)

Die Aldehyde V sind literaturbekannt (vgl. Bull. Chem. Soc. Jap. 45, 916 - 21 (1972); J. Amer. Chem. Soc. 95, 3635 - 45 (1973); Kogyo Kagaku Zasshi 71, 137 - 42 (1968), (C. A. 69, 86 757 m)).

Die zur Synthese der Verbindungen der Formel I außerdem benötigten (Thiono)(Thiol)Phosphor(Phosphon)säureester(amid)halogenide III sind aus Houben-Weyl, Methoden der organischen Chemie, Band XII/2, S. 274 ff, Georg-Thieme-Verlag, Stuttgart, 1964 bekannt und lassen sich auf den dort beschriebenen Synthesewegen herstellen.

Die neuen Verbindungen der Formel I fallen teilweise in Form farbloser oder schwach bräunlich gefärbter Öle an, die sich durch längeres Erwärmen unter vermindertem Druck auf mäßig erhöhte Temperatur ("Andestillieren") von den letzten flüchtigen Anteilen befreien und auf diese Weise reinigen lassen. Erhält man die Verbindungen der Formel I in kristalliner Form, so kann ihre Reinigung durch Umkristallisation erfolgen.

Da die Oximinophosphorsäurederivate der Formel I in den strukturisomeren syn- und anti-Formen auftreten können, eignen sich ibre Schmelz- bzw. Siedebereiche wenig zu ihrer Charakterisierung; zu diesem Zweck dienen daher im folgenden H-NMR-Spektren, Elementaranalyse und IR-Spektren mit typischen Absorptionsmaxima aus dem sog. "fingerprint" Bereich zwischen 1 500 $cm^{-1}$ und 900 $cm^{-1}$.

Die folgenden Beispiele erläutern die Herstellung der Oriminophosphorsäurederivate der Formel I.

**Vorprodukte**

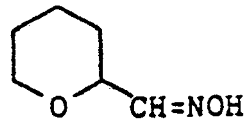

41,8 g gepulvertes Natriumhydroxid werden in einer Mischung aus 100 ml Wssser und 315 ml Ethanol gelöst. Unter Kühlung fügt man 72,6 g Hydroxylaminhydrochlorid in 70 ml Wasser hinzu, saugt vom ausgefallenen Natriumchlorid ab und tropft das Filtrat unter Rühren bei maximal 30°C zu 108,2 g 2-Formyltetrahydropyran. Die Reaktionsmischumg wird 12 Stunden bei Raumtemperatur nachgerührt und in Wasser gegossen. Die wäßrige Phase wird mit Kochsalz gesättigt und sechsmal mit je 100 ml Ether extrahiert. Die vereinigten Extrakte werden über Natriumsulfat getrocknet. Nach dem Abziehen des Lösungsmittels - zuletzt bei 0,05 mbar und 35°C - verbleiben 95,4 g 2-Formyltetrahydropyranoxim als nahezu farbloses Öl.

Ausbeute: 78 % der berechneten Menge.
$C_6H_{11}NO_2$ (129)
ber: C 55,8 H 8,6 N 10,8
gef: C 55,9 H 8,7 N 11,0
Infrarotabsorptionen ($cm^{-1}$): 1 202, 1 085, 1 042, 970, 952, 940
In analoger Weise ist mit vergleichbarer Ausbeute 2-Formyltetrehydrofuranoxim hergestellt worden.
$C_5H_9NO_2$ (115)
ber: C 52,2 H 7,9 N 12,2
gef: C 52,4 H 8,0 N 12,5

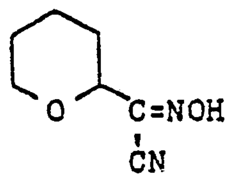

In eine gekühlte Lösung von 12,9 8 2-Formyltetrahydropyranoxim in 140 ml Ether leitet man unterhalb 0°C 7,8 g Chlor ein. Anschließend werden die flüchtigen Bestandteile der Reaktionsmischung abgezogen, der Rückstand in 200 ml Methylenchlorid aufgenormen und 24 Stunden bei Raumtemperatur aufbewahrt. Danach fügt man diese Flüssigkeit tropfenweise zu einer auf 10 bis 15°C abgekühlten Suspension von 6,8 g Kaliumcyanid in 75 ml Methanol und läßt drei Stunden bei Raumtemperatur nachrühren. Das abgeschiedene Kaliumchlorid wird abgesaugt, das Filtrat eingeengt, der Rückstand in Ether aufgenommen, dreimal mit Wasser gewaschen und über Magnesiumsulfat getrocknet. Nach dem Abziehen des Lösungsmittels bei 0,005 mbar und 30°C verbleiben 11,3 g α-Oximino(tetrahydropyran-2-yl)-acetonitril als gelblicher Feststoff, welcher ab 45°C schmilzt.

Ausbeute: 74 % der rechnerisch möglichen.
$C_7H_{10}N_2O_2$ (154)
ber: C 54,6 H 6,6
gef: C 54,8 H 6,8
80-MHz-H-HMR-Spektrum in CDCl3 (δ -Werte in ppm): 140 - 2,20 (m, 6 H); 3,4 - 4,0 (m, 2 H); 4,0 - 4,4 (m, 1 H) 9,8 - 10,2 (breit, 1 H).

**Beispiel 1**

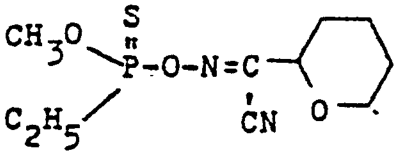

7,7 g +81;Oximino-(tetrahydropyran-2-yl)-acetonitril und 8,6 g Phosphor-säure-0,0-diethylesterchlorid werden in 50 ml Acetonitril vorgelegt und unter Rühren portionsweise mit 3,8 g pulverisiertem Kaliumcarbonat versetzt. Die Reaktionsmischung wird 24 Stunden bei Raumtemperatur gerührt, dann wird von unlöslichen Bestandteilen abgesaugt und das Filtrat im Vakuum eingeengt. Der Rückstand wird in Ether aufgenommen, dreimal mit

Wasser gewaschen, über Natriumsulfat getrocknet und vom Lösungsmittel befreit. Nach dem Andestillieren bei 0,01 mbar und 45°C verbleiben 12,8 g (0,0-Diethylphosphoryl)-α -oximino-(tetrahydropyran-2-yl)acetonitril als nahezu farbloses, viskoses Öl, Ausbeute: 88,5 % der rechnerisch möglichen.

$C_{11}H_{19}N_2O_5P$ (290)
ber: C 45,5 H 6,6
gef: C 45,3 H 6,6
60-MHz-H-HMR-Spektrum in $CDCl_3$ (δ-Werte in ppm): 1,35 (t, 6 H); 1,5 - 2,2 (m, 6 H); 3,4 - 3,9 (m, 2 H); 3,9 - 4,6 (m, 5 H).

**Beispiel 2**

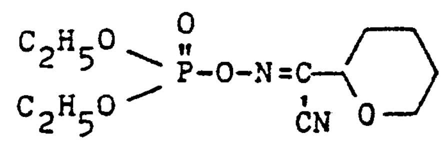

Analog Beispiel 1 erhält man durch Umsetzung von 7,7 g α-Oximino-(tetra-hydropyran-2-yl)acetonitril mit 7,9 g Ethan-thiophosphonsäure-O-methylester-chlorid in Gegenwart von 3,8 g Kaliumcarbonat 12,2 g (89 % der berechneten Menge) (O-Methyl-ethanthiophosphonyl)-α-oximino-(tetrahydro-pyran-2-yl)-acetonitril als gelbliches Öl.

$C_{10}H_{17}N_2O_3PS$ (276)
ber: C 43,5 H 6,2
gef: C 43,6 H 6,4
Infrarotabsorptionen ($cm^{-1}$): 1 086, 1 047, 1 025, 905.

**Beispiel 3**

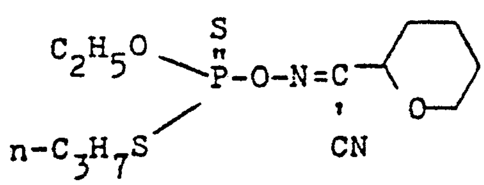

Analog Beispiel 1 werden 7,7 g α-Oximino-(tetrahydropytan-2-yl)aceto-nitril mit 10,9 g Dithiophosphorsäure-O-ethyl-S-n-propyl-diesterchlorid und 3,S g Kaliumcarbonat zur Reaktion gebracht; man erhält 14,0 g (84 % der berechneten Menge) (O-Ethyl-S-n-propyl-dithiophosphoryl)-α-oximino-(tetrahydropyran-2-yl)-acetonitril in Form eines gelben Öles.

$C_{12}H_{21}N_2O_3PS_2$ (332)
ber: C 42,8 H 6,3 N 8,3
gef: C 42,6 H 6,4 N 8,0
Infrarotabsorptionen ($cm^{-1}$): 1 088, 1 047, 1 020, 940, 906

Die nachstehend in der Tabelle 1 aufgeführten verbindungen wurden ebenfalls auf dem in Beispiel 1 beschriebenen Weg erhalten; andere verbindungen, die der Formel (I) entsprechen, können auf die gleiche Weise unter entsprechender Abwandlung der Vorschriften nach der jeweils benötigten Menge und - wegen der besten Reaktionsbedingungen - ggf. nach einem Vorversuch erhalten werden.

Einige typische Verbindungen I, die eine ähnliche biologische Wirkung wie die in Tabelle 1 beschriebenen Verbindungen haben, sind in der Tabelle 2 angegeben.

**Tabelle 1**

| Beispiel Nr. | $R^1$ | $R^2$ | X | n | $^1H$-NMR-Daten (MHz, Lösungsmittel, δ-Werte) oder IR-Absorptionen ($cm^{-1}$). |
|---|---|---|---|---|---|
| 4 | $CH_3$ | $CH_3O$ | S | 2 | (60, $CDCl_3$) 1,4 - 2,2 (m, 6 H); 3,2 - 3,8 (m, 2 H); 3,9 (d, 6 H); 3,9 - 4,5 (m; 1 H) |
| 5 | $C_2H_5$ | $C_2H_5O$ | S | 2 | 1 086, 1 045, 1 020, 940, 903 |
| 6 | $C_2H_5$ | $C_2H_5$ | S | 2 | 1 086, 1 047, 1 020, 1 010, 940, 904 |
| 7 | $C_2H_5$ | NH-i.-$C_3H_7$ | O | 2 | (80, $CDCl_3$) 1,2 (d, 6 H); 1,35 t, 3 H); 1,5 - 2,2 (m, 6 H); 3,0 - 3,8 (m, 3 H); 3,8 - 4,5 (m, 4 H) |
| 8 | $CH_3$ | $CH_3O$ | O | 2 | (80, $CDCl_3$) 1,6 - 2,2 (m, 6 H); 3,4 - 3,9 (m, 2 H); 3,9 (d, 6 H); 3,9 - 4,5 (m, 1 H) |
| 9 | $C_2H_5$ | S-n-$C_3H_7$ | O | 2 | (60, $CDCl_3$) 1,0 (t, 3 H); 1,4 (t, 3 H); 1,4 - 2,2 (m, 8 H); 2,4 - 3,2 (2 t; 2 H); 3,3 - 3,75 (m, 2 H); 3,8 - 4,6 (m, 5 H) |

Tabelle 2

| Beispiel Nr. | $R^1$ | $R^2$ | X | n |
|---|---|---|---|---|
| 10 | $C_2H_5$ | $C_6H_5$ | S | 2 |
| 11 | $C_2H_5$ | S-sec.$C_4H_9$ | S | 2 |
| 12 | $C_2H_5$ | $(CH_3)_2N$ | O | 2 |
| 13 | $C_2H_5$ | $CH_3$ | S | 2 |

Die vorstehend aufgeführten und andere erfindungsgemäßen Wirkstoffe werden auf die für Fhosphorsäureester übliche Weise angewendet. Angaben zur Formulierung, Wirkung und über geeignete Mischungspartner zur Erzielung synergistischer und anderer vorteilhafter Wirkungen können beispielsweise der US-pS 4 320 122 entnommen werden, die anstelle einer vollständigen Beschreibung angegeben ist.

Als Vergleichsmittel wurden die aus der DE-OS 29 52 738 bekannten Verbindungen I und II - Formel nachstehend - gewählt.

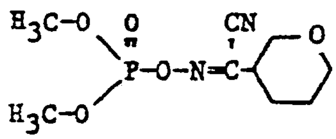

(I)

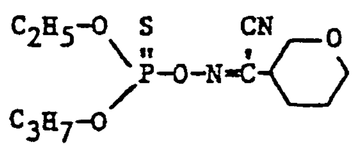

(II)

**Anwendungsbeispiel 1**

**Kontaktwirkung auf Scheben (Blatta orientalis)**

Der Boden eines 1 1-Weckglases wird mit der acetonischen Lösung des Wirkstoffs behandelt° Nach verdunsten des Lösungsmittels setzt man je Glas 5 adulte Schaben. Die Mortalitätsrate wird nach 48 Stunden bestimmt.

In diesem versuch erzielten die Wirkstoffe der Herstellbeispiele 1, 2, 4, 5 und 8 eine bis zu 25fach stärkere Wirkung als die beiden vergleichsmittel.

**Anwendungsbeispiel 2**

**Dauerkontaktwirkung auf Stubenfliegen (Musca domestica)**

Petrischalen mit 10 cm Durchmesser werden innen mit der azetonischen Lösung der Wirkstoffe behandelt. Nach Verdunsten des Lösungsmittels besetzt man die Schalen mit 20-4-Tage alten Stubenfliegen. Die Mortalität wird nach 4 Stunden ermittelt.

Bei diesem Versuch erzielten die Wirkstoffe der Herstellbeispiele 1, 2, 4, 5 und 6 eine bis zu 100fach stärkere Wirkung als das - schwächer wirkende - Vergleichsmittel I und eine bis zu 10fach stärkere Wirkung als die wirksamere Verbindung II.

**Anwendungsbeispiel 3**

**Kontaktwirkung auf Kornkäfer**

Aufgerauhte Glasplatten von 8 x 8 cm Kantenlänge werden mit der acetonischen Wirkstofflösung behandelt. Nach verdunsten des Lösungsmittels belegt man die platten mit 100 Kornkäfern und deckt mit einem Uhrglas (Ø 6 cm) an. Nach 4 Stunden werden die Käfer in unbehandelte Gefäße überführt. Die Mortalitätsrate wird nach 24 Stunden ermittelt. Dabei wird festgestellt, wieviele Käfer in der Lage waren, nach diesem Zeitpunkt innerhalb von 60 Minuten ein unbehandeltes Pappschälchen (Ø 40 mm, Höhe 10 mm) zu verlassen.

Bei diesem Versuch erzielten die Wirkstoffe der Herstellbeispiele 1, 2, 4, 5, 6 und 8 mindestens die gleiche Wirkung wie das - wirksamere - Vergleichsmittel I, während vergleichsmittel II, das eine relativ schwache Wirkung zeigte, um das 200- bis 1000 fache übertroffen wurde.

**Anwendungsbeispiel 4**

**Kontaktwirkung auf Baumwollwanze (Dysdercus intermedius)**

Petrischalen von 10 cm Durchmesser werden mit 1 ml der acetonischen Wirkstofflösung ausgekleidet. Nach Verdunsten des Lösungsmittels besetzt man die Schalen mit je 20 Larven des vorletzten Stadiums und registriert die Wirkung nach 24 Stunden.

Die Wirkstoffe 1, 2, 5 und 6 erwiesen sich bei diesem Versuch als 2 bis 20 mal wirksamer als die Vergleichsmittel 1 und 11.

**Anwendungsbeispiel 5**

**Kohlschabenraupen: Fraß- und Kontaktwirkung (Plutella maculipennis)**

Blätter von jungen Kohlpflanzen werden 3 Sekunden in die wäßrige Wirkstoffemulsion getaucht und nach kurzem Abtropfen auf einen angefeuchteten Filter in eine petrischale gelegt. Das Blatt wird darauf mit 10 Raupen des 4. Stadiums belegt. Nach 48 Stunden beurteilt man die Wirkung.

Bei diesem Versuch erzielten die Wirkstoffe 1, 2, 4, 5, 6 und 7 in bis zu 10 fach geringerer Konzentration als die Vergleichsmittel rund 80 %ige Wirkung.

**Anwendungsbeispiel 6**

**Kontaktwirkung auf Zecken (Ornithodorus moubata)**

Die Prüfung erfolgt an jungen Zecken, die erst einmal Blut aufgenommen haben. Je 5 Tiere werden in einen TEEFIX-Beutel 5 Sekunden in die wäßrige Wirkstoffaufbereitung getaucht. Der Beutel wird frei aufgehängt. Nach 48 Stunden ermittelt man die Mortalitätsrate.

Die Wirkstoffe 1, 5 und 8 erwiesen sich in Verdünnungen von 1 ppm als voll wirksam, während die Vergleichsmittel zur Erzielung vergleichbarer Wirkung in 4 bis 400 fach höherer Konzentration vorliegen müßten.

**Patentansprüche**

1. Oxizinophosphorsäurederivat der Formel 1

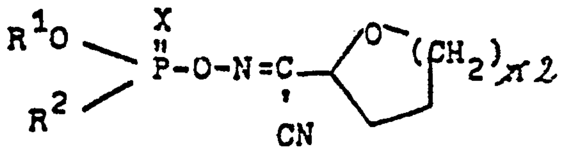

(I),

in der
$R^1$ eine unverzweigte oder verzeigte Alkylgruppe mit bis zu 4 Kohlenstoffatomen,
$R^2$ eine unversweigte oder verzweigte Alkoxy- oder Alkylthiogruppe mit bis zu 4 Kohlenstoffatomen, eine unverzeigte oder verzweigte Alkylgruppe mit bis zu 3 Kohlenstoffatomen, Phenyl, die Aminogruppe oder einen unverzweigten oder verzweigten Alkylamino- oder Dialkylsminorest mit jeweils bis zu 4 Kohlenstoffatomen in einer Alkylgruppe,
X Sauerstoff oder Schwefel bedeuten

2. Verfahren zur Herstellung von Oximinophosphorsäurederivaten der Formel I gemäß Anspruch 1, dadurch gekennzeichnet, daß man ein entsprechendes α-Oximinonitril (II)

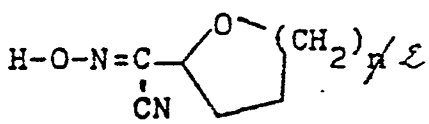

(II),

vorzugsweise in Gegenwart eines Säureacceptors oder Alkalimetall-, Erdalkalimetall- oder gegebenenfalls substituierte Ammoniumsalze dieser α-Oximinonitrile mit einem entsprechenden (Thiono) (Thiol)-Phosphor(Phosphon)säureester(amid)-halogenid (III)

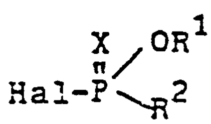

(III),

gegebenenfalls in Gegenwart eines Lösungs- oder Verdünnungsmittels bei Temperaturen zwischen 0 und 120°C umsetzt.

3. Schädlingsbekämpfungsmittel, enthaltend ein Oximinophosphorsäurederivat gemäß Anspruch 1.

4. Schädlingsbekämpfungsmittel, enthaltend einen festen oder flüssigen Trägerstoff und mindestens ein Oximinophosphorsäurederivat der Formel 1 gemäß Anspruch 1.

5. Verwendung von Oxominophosphorsäurederivaten der Formel 1 gemäß Anspruch 1 zur Bekämpfung von Schädlingen.

6. Verfahren zur Bekämpfung von Schädlingen, dadurch gekennzeichnet, daß man eine wirksame Menge des Oximinophosphorsäurederivats gemäß Anspruch 1 auf Schädlinge bzw. deren Lebensraum einwirken läßt.

**Claims**

1. An oximinophosphoric acid derivative of the formula

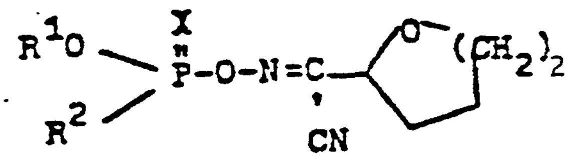

(I),

where

$R^1$ is a straight-chain or branched alkyl group of up to 4 carbon atoms,

$R^2$ is a straight-chain or branched alkoxy or alkylthio group of up to 4 carbon atoms, a straight-chain or branched alkyl group of up to 3 carbon atoms, phenyl, amino, or a straightchain or branched alkylamino or dialkylamino radical where each alkyl is of up to 4 carbon atoms, and

X is oxygen or sulfur.

2. A procees for the preparation of an oximinophosphoric acid derivative of the formule 1 as claimed in claim 1, whereinn an appropriate α-oximinonitrile (II)

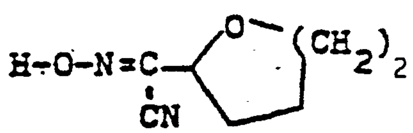

(II),

preferably in the presence of an acid acceptor, or an alkali metal, alkaline earth metal or unsubstituted or substituted ammonium salt of this α-oximinonitrile, is reacted with an appropriate (thiono)(thiol)phosphoric(phosphonic) acid ester (amide) halide

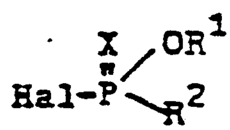

(III)

in the presence or absence of a solvent or diluent at from 0 to 120°C.

3. A pesticide which contains an oximinophosphoric acid derivative as claimed in claim 1.

4. A pesticide which contains a solid or liquid carrier and one or more oximinophosphoric acid derivatives of the formula 1 as claimed in claim 1.

5. Use of an oximinophosphoric acid derivative of the formule I as claimed in claim 1 for controlling pests.

6. A method of controlling pests, wherein an effective amount of an oximinophosphoric acid derivative as claimed in claim 1 is allowed to act on pests or on their habitat.

**Revendications**

1.- Derivé d'acide oximinophoaphorique de formule

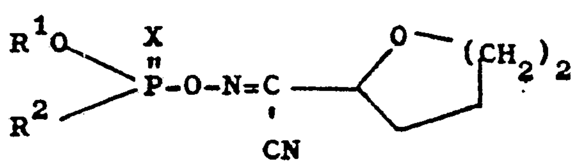

(I)

dans laquelle

$R^1$ représente un groupe alkyle non ramifié on ramifié, ayant jusqu'à 4 atomes de carbone,

$R^2$ représente un groupe alcoxy ou alkylthio non ramifié on ramifié, ayant jusqu'à 4 atomes de carbone, un

groupe alkyle non ramifié ou ramifié, ayant jusqu'à 3 atomes de carbone, phényle, la groupe amino ou un reste alkylamino ou dialkylamino non ramifié ou ramifié, ayant chacun jusqu'à 4 atomes de carbone dans un groupe alkyle,

X étant oxygène ou soufre.

2.- Procédé de preparation de derivés d'acide oximinophosphorique de formule 1 selon la revendication 1, caractérisé par le fait qu'on fait réagir, éventuellement en présence d'un solvant on diluant, à des températures comprises entre 0 ét 120°C, un α-oximinonitrile (II) approprié

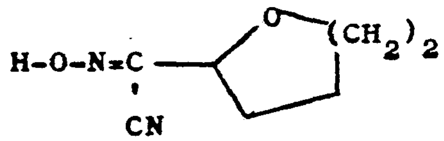 (II)

de préférence en présence d'un accepteur d'acide, ou des sels métalliques alcalins, alcalino-terreux ou des sels d'ammonium, éventuellement substitués, desdits α-oximino-nitriles, avec un (amido)-halogénure d'ester d'acide (thiono) (thiol)-phosphorique (phosphonique)

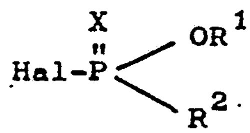 (III)

3.- Pesticides contenant un dérivé d'acide oximinophosphorique selon la revendication 1.

4.- Pesticides contenant un support solide ou liquide et au moins un dérivé d'acide oximinophosphorique de formule 1 selon la revendication 1.

5.- Utilisation de dérivés d'acide oximinophosphorique de formule 1, selon la revendication 1, pour la lutte contre les parasites.

6.- Procédé de lutte contre les parasites, caractérisé par le fait qu'on fait agir une quantité efficace du dérive d'acide oximinophosphorique selon la revendication 1 sur les parasites ou leur biotope.